## Europäisches Patentamt
## European Patent Office
## Office européen des brevets

(19)

(11) Veröffentlichungsnummer: **0 080 074 B1**

(12) **EUROPÄISCHE PATENTSCHRIFT**

(45) Veröffentlichungstag der Patentschrift: 03.04.91

(51) Int. Cl.⁵: **A61K 6/04**

(21) Anmeldenummer: 82110008.8

(22) Anmeldetag: 29.10.82

(54) **Verwendung einer Kobalt-Chrom-Legierung als Werkstoff für Prothesengerüste, die mit dentaler Keramik beschichtet werden.**

(30) Priorität: 20.11.81 DE 3145945

(43) Veröffentlichungstag der Anmeldung:
01.06.83 Patentblatt 83/22

(45) Bekanntmachung des Hinweises auf die
Patenterteilung:
03.04.91 Patentblatt 91/14

(84) Benannte Vertragsstaaten:
AT CH FR GB IT LI

(56) Entgegenhaltungen:
EP-A- 0 041 938
DE-A- 1 558 448
DE-A- 2 025 614
DE-A- 2 511 745

(73) Patentinhaber: **Fried. Krupp Gesellschaft mit beschränkter Haftung**
**Altendorfer Strasse 103**
**W-4300 Essen 1(DE)**

(72) Erfinder: **Becker, Hans-Joachim**
**Martha-Strasse 11**
**W-4300 Essen 1(DE)**
Erfinder: **Friedrich, Ronald**
**Monterkampweg 43**
**W-4132 Kamp-Lintfort(DE)**
Erfinder: **Müller, Manfred**
**Obere Fuhr 57**
**W-4300 Essen 1(DE)**

(74) Vertreter: **Vomberg, Friedhelm, Dipl.-Phys.**
**Schulstrasse 8**
**W-5650 Solingen 1(DE)**

## Beschreibung

Verwendung einer nickelfreien Kobalt-Chrom-Legierung, die aus 15 bis 25 % Chrom, 3 bis 6 % Molybdän 2 bis 5 % Titan, Rest Kobalt einschließlich herstellungsbedingter Verunreinigungen besteht, zur Herstellung eines Prothesengerüstes, das mit einer dentalen Keramik beschichtet wird.

Die Erfindung betrifft die Verwendung einer an sich bekannten Kobalt-Chrom-Legierung, bestehend aus 10 bis 35 % Cr, 1 bis 10 Ti, 2 bis 15 % Mo, Rest Kobalt einschließlich herstellungsbedingter Verunreinigungen als Werkstoff für die Dentalprothetik.

Es ist bekannt, daß Kobalt-Chrom-Legierungen der obengenannten Zusammensetzung hohen korrosiven, mechanische und/oder thermischen Belastungen widerstehen können. So wird beispielsweise als Werkstoff für Dampf- und Gasturbinen nach "Technischen Mitteilungen des Hauses der Technik, Essen" 1968, Seite 554 eine Kobalt-Chrom-Legierung mit 20 bis 33 % Chrom vorgeschlagen. Darüber hinaus ist nach der DE-A-25 11 745 eine solche Legierung auch als Werkstoff für Zahnprothesen und chirurgische Implantate geeignet. Insbesondere werden die genannten Kobalt-Chrom-Legierungen als Dentalgußlegierungen für den herausnehmbaren Zahnersatz verwendet.

Dagegen bedient man sich bei festsitzendem Zahnersatz, der mit in der Dentalprothetik bekannten Keramiken verblendet wird, z.B. bei Kronen und Brücken, größtenteils Legierungen auf Gold-Platinbasis bzw. Legierungen, bei denen Gold und Platin teilweise oder auch ganz durch Silber und Palladium ersetzt worden ist, oder hochnickelhaltiger Legierungen. Die genannten Edelmetallegierungen wie auch die Nickellegierungen haben bekanntlich einen in etwa den Dentalkeramiken entsprechenden thermischen Ausdehnungskoeffizienten, so daß es beim Aufbrennen der Keramik auf die Legierung des Grundkörpers bei keiner der Temperaturen, denen das Material ausgesetzt werden muß, zu Spannungen oder Rißbildungen führt. Allerdings sind die Gold-Palladium-Basislegierungen sehr kostspielig und die Nickel-Chrom-Legierungen gewebe- bzw. hautunverträglich. Darüber hinaus ist bekannt, daß Nickel zu den karzinogenen Stoffen zählt, so daß dessen Verarbeitung gesundheitsgefährdende Risiken schafft.

Es ist daher Aufgabe der Erfindung, eine Legierung zu finden, die möglichst nickelfrei ist und die sich als Aufbrennlegierung, d.h. zum Beschichten (Verblenden) mit Keramiküberzügen eignet. Darüber hinaus soll diese Legierung möglichst preiswert sein und als Ersatzmaterial für die bisher bekannten Edelmetallegierungen dienen.

Bei der Suche nach einer geeigneten Legierung mußte der Fachmann bisher davon ausgehen, daß die bekannten Legierungen auf Kobalt-Chrom-Basis im Temperaturintervall von Raumtemperatur bis zu 600 °C thermische Ausdehnungskoeffizienten von mehr als $15 \times 10^{-6}$ $K^{-1}$ besitzen. Legierungen mit solch hohen Ausdehnungskoeffizienten sind jedoch nicht geeignet, mit Keramik einen Verbundkörper einzugehen, da aufgrund der dann vorliegenden unterschiedlichen thermischen Ausdehnungskoeffizienten leicht Zerstörungen des Verbundkörpers durch Rißbildung in der Keramik oder Abplatzung vom Metall eintreten können.

Überraschenderweise hat sich jedoch herausgestellt, daß die an sich bereits bekannte nickelfreie Kobalt-Chrom-Legierung aus 10 bis 35 % Cr, 1 bis 10 Ti, 2 bis 15 % Mo, Rest Kobalt hervorragend zur Verblendung mit Keramik geeignet ist. Messungen des linearen thermischen Ausdehnungskoeffizienten haben den bisher nicht vermuteten Wert von weniger als $15 \times 10^{-6}$ $K^{-1}$ ergeben.

Bevorzugt verwendet man jedoch Legierungen, die im wesentlichen aus 2 bis 5 % Titan, 3 bis 6 % Molybdän, 15 bis 25 % Chrom, Rest Kobalt bestehen. Insbesondere, wenn die Legierung nicht unter Vakuum vergossen werden soll, empfiehlt es sich nach einer weiteren Ausgestaltung der Erfindung, 0,1 bis 3 % Mangan, 0,1 bis 2 % Eisen, 0,1 bis 2 % Aluminium, und/oder 0,01 bis 1 % Silizium der Legierung zuzusetzen. Hierbei wirken Mangan und Silizium als hervorragend geeignete Desoxidationsmittel.

Vorteilhafterweise wird allerdings unter Vakuum vergossen, wodurch eine größtmögliche Reinheit der Legierung erreichbar ist.

Ebenso wie die nach dem Stand der Technik bekannten Edelmetallegierungen kann die erfindungsgemäß vorgeschlagene Legierung auch mit anderen in der Zahntechnik üblichen Gußverfahren erschmolzen und abgegossen und vor dem Keramikbrand einer Oxidglühung unterzogen werden. Die erfindungsgemäße Legierung ist gegenüber den bisher hauptsächlich verwendeten Edelmetallegierungen nicht nur preisgünstiger, sondern zeichnet sich auch durch eine höhere Festigkeit, einen höheren Schmelzpunkt, eine geringere Dichte und eine geringere Wärme leitfähigkeit aus. Durch den höheren Schmelzpunkt und die größere Festigkeit auch bei höheren Temperaturen, wird eine unbeabsichtigte Verformung der gegossenen Teile beim Keramikbrand, wie sie z.B. bei Edelmetallegierungen die zu größeren Prothesen verarbeitet werden sollen, allein durch die Eigengewichtsbelastung schon auftreten kann, vermieden. Durch die geringe Dichte der erfindungsgemäßen Legierung wird gegenüber den Edelmatallegierungen Gewicht gespart, was die herzustellenden Prothesen weiter verbilligt. Die geringe Wärmeleitfähigkeit der erfindungsgemäßen Legierung wirkt sich insbesondere an präparierten Zahnstümpfen, die empfindlich auf rasche Temperaturwechsel reagieren, vorteilhaft aus.

Die Erfindung wird nachfolgend anhand von Ausführungsbeispielen, deren chemische Zusammensetzungen in Tabelle 1 angegeben werden, näher erläutert.

## Tabelle 1

| Beispiel Nr. | Ti % | Mo % | Cr % | Co % | Härte HB |
|---|---|---|---|---|---|
| 1 | 2 | 5 | 20 | Rest | 235 |
| 2 | 3 | 3 | 20 | Rest | 260 |
| 3 | 3 | 3 | 25 | Rest | 265 |
| 4 | 3 | 4 | 20 | Rest | 265 |
| 5 | 3 | 5 | 20 | Rest | 270 |
| 6 | 4 | 3 | 20 | Rest | 275 |
| 7 | 4 | 5 | 20 | Rest | 310 |
| 8 | 5 | 5 | 15 | Rest | 330 |

Die Tabelle 1 gibt auch Aufschluß über die Brinellhärte der dort angegebenen Legierungen.

Zwei der in Tabelle 1 angegebenen Legierungen sind auf ihre weiteren mechanischen Eigenschaften untersucht worden. Die in Tabelle 2 wiedergegebenen mechanischen Eigenschaften wurden an Proben im Gußzustand ermittelt. Die erfindungsgemäße Legierung zeichnet sich demnach insbesondere durch günstige lineare thermische Ausdehnungskoeffizienten um $14 \times 10^{-6}$ $K^{-1}$ aus, die in der Größenordnung der linearen thermischen Ausdehnungskoeffizienten für Keramiken liegen. Darüber hinaus besitzt die Legierung bekanntlich eine hohe Zugfestigkeit sowie hohe 0,2-Dehngrenzen und hohe Bruchdehnungen. Diese hohe Duktilität wirkt sich vorteilhaft bei der Verarbeitung von komplizierten Formen aus. Schließlich ist aus elektrochemischen Untersuchungen bekannt, daß die in Tabelle 1 angegebenen Legierungen eine hervorragende Beständigkeit gegenüber gleichmäßiger Flächenkorrosion und Lochkorrosion besitzen. Für medizinische Anwendungen ist dabei besonders die sehr hohe Beständigkeit gegenüber Spaltkorrosion hervorzuheben.

## Tabelle 2

| Beispiel Nr. | $R_{pO,2}$ $N/mm^2$ | $R_m$ $N/mm^2$ | $A_5$ % | $10^{-6}$ $°C^{-1}$ |
|---|---|---|---|---|
| 5 | 530 | 700 | 10 | 14,5 |
| 8 | 795 | 1070 | 15,4 | 13,5 |

EP 0 080 074 B1

### Ansprüche

1. Verwendung einer nickelfreien Kobalt-Chrom-Legierung, die aus 15 bis 25 % Chrom, 3 bis 6 % Molybdän, 2 bis 5 % Titan, Rest Kobalt einschließlich herstellungsbedingter Verunreinigungen besteht, zur Herstellung eines Prothesengerüstes, das mit einer dentalen Keramik beschichtet wird.

2. Verwendung einer Legierung nach Anspruch 1, die unter Vakuum vergossen worden ist.

### Claims

1. Use of a nickel-free cobalt-chromium alloy consisting of 15 to 25% chromium, 3 to 6% molybdenum, 2 to 5% titanium, residue cobalt, including impurities caused by production, for making a prosthesis structure which is coated with a dental ceramic.

2. Use of an alloy according to claim 1, which has been cast in vacuo .

### Revendications

1. Utilisation d'un alliage cobalt-chrome, sans nickel, constitué de 15 à 25 % de chrome, 3 à 6 % de molybdène, 2 à 5 % de titane, le reste de cobalt, y compris des impuretés causées par la fabrication, pour la réalisation d'une construction prothétique, qui est revêtue d'une céramique dentaire.

2. Utilisation d'un alliage suivant la revendication I, qui a été coulé sous vide.

4